(19) Europäisches Patentamt
European Patent Office
Office européen des brevets

(11) Numéro de publication: **0 217 719**
**A2**

# (12) DEMANDE DE BREVET EUROPEEN

(21) Numéro de dépôt: 86402197.7

(22) Date de dépôt: 03.10.86

(51) Int. Cl.⁴: **C 07 C 149/18**
**C 07 C 148/00, A 23 L 1/226**

(30) Priorité: 03.10.85 FR 8514680

(43) Date de publication de la demande:
08.04.87 Bulletin 87/15

(84) Etats contractants désignés:
CH DE GB LI

(71) Demandeur: INSTITUT NATIONAL DE LA RECHERCHE AGRONOMIQUE, Etablissement public dit:
145, Rue de l'Université
F-75341 Paris Cédex 07(FR)

(72) Inventeur: Rigaud, Jacques
230 rue de la Fontainette Prades-Le-Lez
F-34980 Saint Gely Du Fesc(FR)

(72) Inventeur: Etievant, Patrick
248 rue de Talant
F-21000 Dijon(FR)

(72) Inventeur: Henry, Robert
29 rue de Créqui
F-69006 Lyon(FR)

(72) Inventeur: Latrasse, Alain
11 route de Paris
F-21370 Plombieres Les Dijon(FR)

(74) Mandataire: Phélip, Bruno et al,
c/o Cabinet Harlé & Phélip 21, rue de la Rochefoucauld
F-75009 Paris(FR)

## (54) Nouveau dérivé du butanethiol entrant dans la constitution de l'arôme du bourgeon de cassis, ses procédés de préparation et application de ce dérivé à titre d'arôme.

(57) Le nouveau composé est représenté par la formule (I)

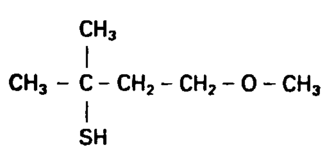

(I)

Il peut être extrait du bourgeon de cassis ou synthétisé directement en faisant réagir le magnésium sur un halogénure d'alkyle (II)

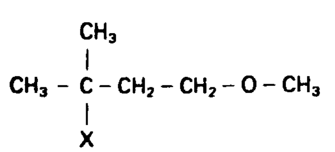

(II),

pour obtenir un composé organomagnésien, sur lequel on fait réagir le soufre, et on hydrolyse le composé obtenu en milieu faiblement acide. Application à titre d'arome ou d'agent renforçateur d'arôme.

EP 0 217 719 A2

La présente invention concerne, d'une part, un nouveau composé chimique consistant en un butanethiol substitué, ce composé étant un constituant de l'arôme du bourgeon de cassis, d'autre part, deux procédés d'obtention de ce composé, le premier mettant en oeuvre une extraction à partir de bourgeons de cassis qui conduit à l'huile essentielle et qui est suivie de chromatographies préparatives, et le second consistant en une synthèse, et, enfin, l'utilisation de ce nouveau composé à titre d'arôme.

Jusqu'à présent, on n'avait pas réussi à isoler et identifier un constituant, qui semblait important, pour l'arôme du bourgeon de cassis.

On sait que l'essence du bourgeon de cassis possède des propriétés aromatiques intéressantes. Elle est utilisée dans les industries alimentaires comme renforçateur de l'arôme du fruit. Elle renferme, pour 85%, des hydrocarbures mono- et sesquiterpéniques, dont nombre d'entre eux ont été identifiés.

L'essence de bourgeon de cassis dégage une odeur puissante, différente de celle du fruit mûr, où prédominent une note "résineux" - dont on sait que ce sont les monoterpènes qui en sont en grande partie responsables -, et surtout la note "urine de matou". Or, c'est précisément le composé qui possède une odeur agressive rappelant celle de l'urine de matou qui n'avait pu être identifié jusqu'ici.

Le Déposant a donc cherché à isoler ce constituant, dont les propriétés aromatiques pouvaient s'avérer très intéressantes.

Au cours de travaux préliminaires, le Déposant a observé que l'addition d'acétate de cuivre à une macération hydro-alcoolique de bourgeons de cassis supprimait la note "urine de matou", ce qui indiquait l'existence possible d'un thiol. Le Déposant a également analysé, par chromatographie en phase gazeuse, un espace de

tête de bourgeons de cassis selon la technique décrite par QVIST I.H. et VON SIDOW E. dans "J. Agr. Food Chem., 24, 437 (1976)". L'utilisation d'un détecteur à photométrie de flamme a permis de mettre en évidence des composés soufrés. Ayant comparé leur volume de rétention avec celui de témoins, le Déposant a émis l'hypothèse que ces composés pouvaient être le méthanethiol, le sulfure de méthyle, le sulfure d'éthyle et de méthyle, le disulfure de diméthyle et le dithia-2,4 pentane. Cependant, aucun de ces composés ne pouvaient donner la note "urine de matou". Comme on pouvait donc conclure que la teneur du produit responsable était très faible dans le bourgeon de cassis, le Déposant a dû recourir à diverses techniques d'enrechissement et de piégeage pour isoler le composé recherché.

Ainsi, en fractionnant l'huile essentielle de bourgeon de cassis par chromatographie liquide-solide pour éliminer les hydrocarbures mono- et sesquiterpéniques, et en faisant subir à la (ou aux) fraction(s) restante(s) des chromatographies en phase gazeuse préparatives successives, le Déposant a pu isoler le composé recherché, dont il a déterminé la structure, ce composé est effectivement un nouveau thiol. Ce thiol a alors pu être synthétisé, les résultats des analyses du composé obtenu se corroborant avec les précédents pour l'identification dudit composé, lequel a donc été déterminé comme étant le méthoxy-4 méthyl-2 butanethiol-2, pouvant être représenté par la formule (I) suivante:

$$CH_3-\underset{\underset{SH}{|}}{\overset{\overset{CH_3}{|}}{C}}-CH_2-CH_2-O-CH_3 \qquad (I)$$

Outre ce nouveau composé de formule (I), la présente invention a pour objet un premier procédé

permettant de le préparer. Conformément à ce premier procédé:

- dans une première étape, on prépare de l'huille essentielle de bourgeon de cassis;

- dans une seconde étape, on réalise le fractionnement de ladite huile essentielle, par chromatographie liquide-solide, en au moins deux fractions, la première qui renferme les hydrocarbures mono- et sesquiterpéniques entrant dans la composition de ladite huile essentielle et que l'on obtient en utilisant, comme éluant, un milieu solvant apolaire, et la seconde, ou chacune des fractions suivantes, que l'on obtient en utilisant comme éluant, respectivement un milieu solvant polaire, ou successivement des milieux solvants de polarité croissante;

- dans une troisième étape, on réalise une première chromatographie en phase gazeuse d'au moins une partie de la (ou des) fraction(s) obtenue(s) à la seconde étape qui ne renferme(nt) pas lesdits hydrocarbures, en pratiquant au moins une injection dans l'appareil de chromatographie et en piégeant, à chaque fois, à la sortie dudit appareil, la portion où prédomine la note "urine de matou". Le composé est récupéré à l'aide d'un milieu solvant à caractère polaire, par élution;

- dans une quatrième étape, on réalise une seconde chromatographie en phase gazeuse d'au moins une partie de l'éluat obtenu à la troisième étape, en pratiquant au moins une injection dans l'appareil de chromatographie et en piégeant, à chaque fois, à la sortie dudit appareil, la portion correspondant au composé recherché, et on recueille ledit composé à l'état pur.

Conformément à un mode de réalisation préféré de ce premier procédé, à la seconde étape, on réalise le fractionnement de l'huile essentielle en trois fractions.

On préfère, à la seconde étape, utiliser le pentane comme milieu solvant apolaire ; dans le cas où l'on obtient deux fractions, le dichlorométhane ou un mélange pentane-dichlorométhane comme milieu solvant polaire ; et, dans le cas où l'on obtient un nombre de fractions supérieur à deux, un (ou des) mélange(s) pentane-dichlorométhane et le dichlorométhane comme milieux solvants de polarité croissante. Dans le cas d'un fractionnement de l'huile essentielle conduisant à trois fractions, on préfère utiliser, comme milieu solvant pour obtenir la deuxième fraction, un mélange pentane-dichlorométhane, dans un rapport en volume de 80-20.

De préférence, à la seconde étape, on utilise une colonne de chromatographie liquide-solide dont l'adsorbant est constitué par de la silice.

On préfère utiliser, à la troisième étape, le même milieu solvant polaire que celui utilisé à la seconde étape dans le cas où l'on obtient deux fractions, ou que l'un des milieux solvants utilisés à ladite seconde étape dans le cas où l'on obtient un nombre de fractions supérieur à deux.

A la première étape, on prépare généralement l'huile essentielle de bourgeon de cassis en effectuant un entraînement à la vapeur d'eau, suivi d'une extraction au pentane.

La présente invention a également pour objet un second procédé permettant de préparer le composé de formule (I), ce second procédé étant caractérisé par le fait que :

- dans une première étape, on fait réagir, en solution inerte et sous atmosphère inerte, le magnésium avec un halogénure d'alkyle de formule (II) :

$$CH_3-\underset{\displaystyle X}{\overset{\displaystyle CH_3}{\underset{|}{\overset{|}{C}}}}-CH_2-CH_2-O-CH_3 \qquad (II)$$

  formule dans laquelle X est choisi parmi le chlore, le brome et l'iode,

pour obtenir le composé organo-magnésien correspondant de formule (III) :

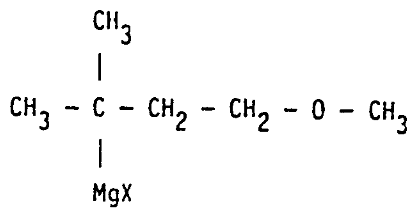 (III)

- dans un seconde étape, on fait réagir le soufre sur le composé de formule (III) pour obtenir le composé de formule (IV) :

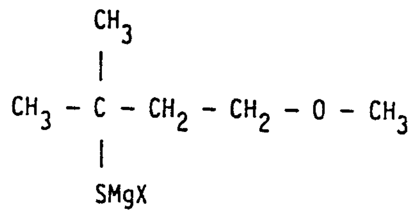 (IV)

- dans une troisième étape, on hydrolyse le composé de formule (IV) en milieu faiblement acide.

On utilise de préférence un composé de formule (II), dans laquelle X est le chlore.

De préférence également, à la première étape de ce second procédé, on prépare le composé de formule (III) en solution dans l'éther anhydre.

Le composé de formule (II), pour lequel X est le chlore, est avantageusement préparé par réaction d'isobutylène et de chlorométhyléther en présence de chlorure mercurique.

La présente invention a enfin pour objet l'utilisation du composé de formule (I) à titre d'arôme ; elle porte également sur des compositions destinées à être ingérées, caractérisées par le fait qu'elles renferment le composé de formule (I) à titre d'arôme ou d'agent renforcateur d'arôme. La particularité de ce composé à titre d'arôme est que son seuil de perception olfactive est particulièrement bas, de l'ordre de $10^{-6}$ p.p.m. dans l'eau, et de l'ordre de $10^{-4}$ p.p.m. dans des liqueurs. La présente invention porte par conséquent, en particulier, sur des compositions aqueuses, caractérisées par le fait que leur teneur minimale en

composé de formule (I) est de l'ordre de $10^{-6}$ p.p.m., ainsi que sur des liqueurs, caractérisées par le fait que leur teneur minimale en composé de formule (I) est de l'ordre de $10^{-4}$ p.p.m.

Pour illustrer encore la présente invention, on décrira plus en détail ci-après, à titre indicatif et non limitatif, des modes de réalisation particuliers des premier et second procédés définis ci-dessus; on décrira également les analyses qui ont été faites du composé selon l'invention ainsi obtenu, en référence aux dessins annexés sur lesquel les Fig. 1 à 3 représentent les spectres respectivement de masse, infra-rouge dans le tétrachloréthylène, et de résonance magnétique nucléaire dans le benzène deutérié;

On décrira enfin différents tests pour la détermination du seuil de perception olfactive du composé obtenu.

A - OBTENTION DU COMPOSE DE FORMULE (I) PAR LE PREMIER PROCEDE

a) Obtention de l'huile essentielle:

Des bourgeons de cassis, appartenant à la variété "NOIR DE BOURGOGNE" avaient été congelés et conservés pendant 6 mois à -18°C. L'huile essentielle a été obtenue, à partir de 200 g de bourgeons, par un entraînement à la vapeur d'eau, selon ce qui est pratiqué couramment dans l'industrie, et une extraction au pentane, en continu, pendant une heure, dans un appareil de LIKENS et NICKERSON.

b) Fractionnement de l'huile essentielle:

Après concentration, l'extrait a été fractionné sur une colonne de gel de silice "KIESELGEL 80" (MERCK), ledit gel ayant été activé pendant 14 heures, à 120°C, puis réhydraté à raison de 5 ml d'eau pour 15 g de silice. L'élution a été effectuée par trois volumes de 50 ml chacun de pentane, pentane-dichlorométhane (dans un

rapport en volume 80-20) et dichlorométhane pur, qui ont conduit respectivement aux fractions I, II et III. La fraction I renfermait les hydrocarbures mono- et sesquiterpéniques; la fraction II, la plus grande partie du composé qui était recherchée, celui-ci se retrouvant aussi, mais en moindre proportion, dans la fraction III, du fait qu'il tend à s'adsorber sur la silice.

c) Chromatographies en phase gazeuse préparatives:

1. On commence par réaliser une chromatographie de la fraction II sur une colonne de diamètre extérieur de 3,175 mm d'huile de silicone apolaire "SE 30" à 10% sur une poudre de diatomées "CHROMOSORB W" d'une granulométrie de 0,177-0,147 mm traitée au diméthyldichlorosilane pour l'inactiver. Cette chromatographie est effectuée dans les conditions de température suivantes: 80 à 200°C, à raison de 6°C par minute. Au cours d'une dizaine d'injections (20 µl à chaque fois), on a piégé, dans un petit tube de verre contenant 10 mg d'un support poreux de chromatographie "PORAPAK Q", la partie sortant de l'appareil de chromatographie où a été repérée la note "urine de matou", caractéristique du composé recherché. La désorption du piège a été réalisée en trois fois par 60 µl de dichlorométhane au total.

2. Cet éluat a été soumis à une seconde chromatographie sur une colonne en verre de type capillaire, de longueur 25 m, de diamètre intérieur 1 mm, renfermant un adsorbant en poudre recouvrert par un film de FFAP (Free Fatty Acid Phase), d'une épaisseur de 0,8 µm. Le gaz vecteur était l'helium, avec un débit de 15 ml/mn et la température programmée de 80 à 160°C à 6°C/mn. Le piégeage a été effectué dans un tube de verre en forme de U, plongé dans l'azote liquide. On a recueilli une quantité de l'ordre de 0,1 mg du produit attendu, lors de chacune des deux injections effectuées, ce qui a permis de

tracer les spectres infrarouge et de résonance magnétique nucléaire, comme cela sera décrit plus loin.

B - <u>OBTENTION DU COMPOSE DE FORMULE (I) PAR LE DEUXIEME PROCEDE (SYNTHESE)</u>

a) <u>Préparation du méthoxy-4 chloro-2 méthyl-2 butane</u>:

Le schéma réactionnel est le suivant:

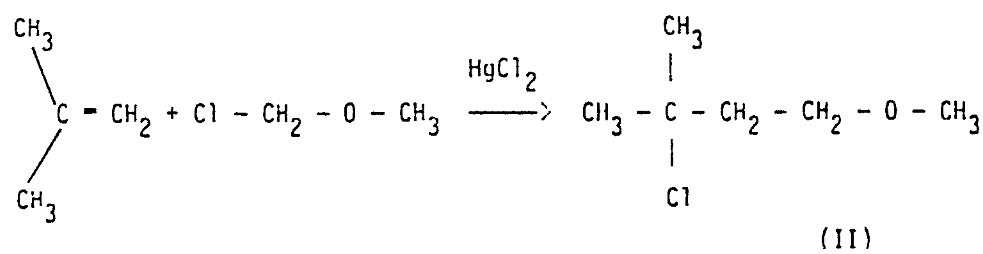


(II)

50 ml d'isobutylène liquide (0,6 mole), 40 g de chlorométhyléther (0,5 mole) et 2 g de chlorure mercurique sont placés dans une bombe hermétiquement fermée. Le mélange est agité pendant une heure, puis laissé au repos pendant deux jours. La solution est distillée sous léger vide. La fraction distillant entre 50 et 80°C sous 50 mm Hg est recueillie, puis redistillée. Ce mode opératoire est conforme à celui indiqué par STRAUS W. et THIEL G. dans "Ann. Chem., <u>525</u>, 151 (1936)".

On obtient 28 g du produit attendu, lequel présente un point d'ébullition de 65°C sous 5 333 Pa (40 mm Hg).

b) <u>Synthèse du méthoxy-4 méthyl-2 butanethiol-2</u>:

Le schéma réactionnel est le suivant:

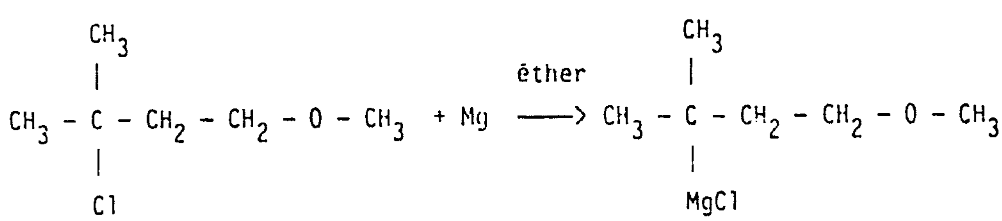

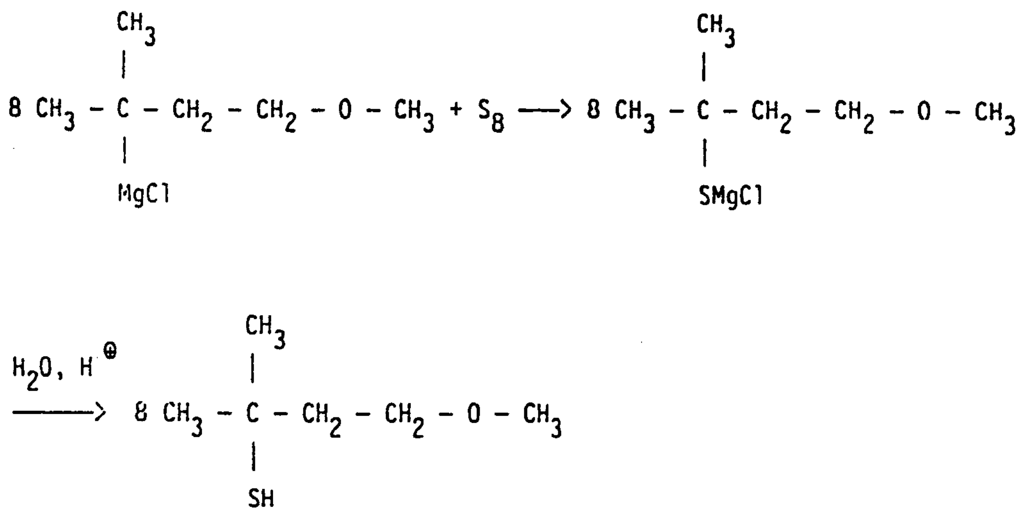


(I)

Dans un ballon tricol de 500 ml, muni d'un réfrigérant et d'une ampoule de coulée, sous atmosphère d'azote, on place 9 g de magnésium en fins copeaux recouverts de 50 ml d'éther anhydre. On ajoute 1 ml de méthoxy-4 chloro-2 méthyl-2 butane et un cristal d'iode; après démarrage de la réaction, on ajoute une solution de 27,7 g de chloro-2 méthoxy-4 méthyl-2 butane dans 100 ml d'éther anhydre, en 5 heures, sous agitation à la température ambiante. A la fin de l'addition, on maintient l'agitation pendant une heure. On décante la solution de magnésien, on mesure son volume et on le dose avec de l'acide sulfurique N. On ajoute ensuite le soufre par petites quantités, dans le rapport de 0,8 atome-gramme de soufre par mole de magnésien. Il faut 3,2 g (0,1 atome-gramme) de soufre, qui sont ajoutés en une heure sous forte agitation. Après une nuit de repos, on hydrolyse d'abord à l'eau froide, puis à l'acide sulfurique dilué. Après décantation, extraction à l'éther, séchage sur sulfate de sodium, la distillation sous léger vide fournit

4,5 g de méthoxy-4 méthyl-2 butanethiol-2 pur. Le point d'ébullition de ce composé est de 74°C sous 5 333 Pa (40 mm Hg).

C - ANALYSE SPECTROSCOPIQUE DU COMPOSE DE FORMULE (I) OBTENU PAR LE PREMIER PROCEDE SUS-INDIQUE:

a) Spectrométrie de masse:

On a réalisé un couplage chromatographie en phase gazeuse-spectrométrie de masse. Le chromatographe en phase gazeuse utilisé était équipé d'une colonne capillaire en verre de 0,5 mm de diamètre et de 50 m de longueur, la phase stationnaire étant un polyéthylèneglycol vendu sous la dénomination "CARBOWAX 20 M", le gaz vecteur étant de l'hélium avec un débit de 5 ml/mm. Ce chromatographe était couplé avec un spectromètre de masse quadripolaire de type "RIBERMAG R 10-10". L'énergie des ions était de 70 eV et la température de la source de 120°C. Le spectre obtenu est représenté sur la Fig. 1.

Un spectre, non représenté sur le dessin, a aussi été tracé en ionisation chimique avec l'ammoniac comme gaz réactant.

b) Spectrométrie infrarouge

On a utilisé, pour tracer le spectre infrarouge représenté sur la Fig. 2, un appareil "PERKIN ELMER 857", équipé d'un condenseur de faisceau. La même quantité de produit, en solution dans le tétrachloréthylène, a été introduite dans une microcellule de NaCl d'une capacité de 20 µl pour un trajet optique de 1 mm.

c) Spectrométrie de résonance magnétique nucléaire:

On a utilisé un spectromètre "RMN BRUKER WP 80"; le composé (0,1 mg environ) était dissous dans le benzène deutérié.

d) Résultats et discussion des résultats

Le spectre de masse indique, comme masse moléculaire, la valeur de 134, ce qui a été confirmé par l'ionisation chimique qui montre aussi la présence d'un

atome de soufre par les abondances relatives des ions 137/135 et 154/152. La perte d'un fragment neutre de masse 34 à partir de l'ion moléculaire laisse supposer l'existence d'un groupement -SH. Cette hypothèse est vérifiée par la présence d'une bande faible à 2580 $cm^{-1}$ sur le spectre infra-rouge. Celui-ci montre l'absence de groupements hydroxyle et d'insaturation carbonée dans la molécule, ainsi que la présence possible d'un groupement méthoxyle (bande à 2820 $cm^{-1}$). Celui-ci correspondrait au singulet d'intensité 3 observé à 3,06 p.p.m., sur le spectre de résonance magnétique nucléaire. Ce spectre présente 4 signaux (t/2 à 3,37 p.p.m., s/3 à 3,06 p.p.m., m/3 à 1,65 p.p.m., s/6 à 1,20 p.p.m.), imputables à la présence, dans la molécule, de 14 atomes d'hydrogène. Compte-tenu de la présence d'un atome de soufre et d'un atome d'oxygène, la formule brute serait donc $C_6H_{14}OS$. Le spectre de résonance magnétique nucléaire montre, outre un groupement méthoxyle, la présence de deux groupements méthylène (S à 1,20 p.p.m.), magnétiquement équivalents, portés par un carbone tertiaire. Les deux massifs restants sont couplés entre eux, car un découplage homonucléaire produit par une irradiation centrée à 3,37 p.p.m. résout le multiplet à 1,65 p.p.m. en deux singulets. Ce multiplet est donc probablement formé par la superposition d'un triplet d'intensité 2, centré à 1,74 p.p.m. et d'un singulet à 1,57 p.p.m.. Ces éléments sont en parfait accord avec la structure suivante :

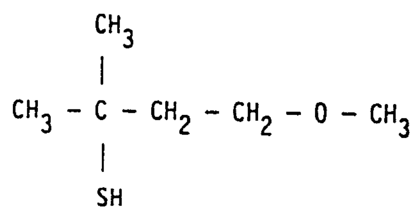

Cette hypothèse a été vérifiée par la synthèse originale de la molécule (deuxième procédé présentement décrit), dont les spectres de masse et infra-rouge, ainsi que le volume de rétention en chromatographie en phase gazeuse sont identiques à ceux du composé obtenu par le premier procédé, composé que l'on cherchait à identifier.

D - <u>DETERMINATION DU SEUIL DE PERCEPTION OLFACTIVE DU COMPOSE SELON L'INVENTION</u> :

La pureté du composé de synthèse piégé (obtenu par le deuxième procédé de l'invention) a été vérifiée par chromatographie en phase gazeuse sur une colonne capillaire de polyéthylèneglycol "CARBOWAX 20 M" ; un contrôle olfactif parallèle à l'enregistrement du chromatogramme, effectué de facon permanente durant toute l'analyse, a montré qu'il n'y avait pas d'autres odeurs notables, donc pas d'impuretés susceptibles de fausser la mesure de la valeur du seuil.

Un jury, constitué de 10 personnes non spécialement entrainées, a procédé à six séances de dégustation étalées sur quatre jours. Pour la réalisation des tests triangulaires et l'interprétation des résultats, on a procédé comme décrit par SALO P. dans "J. Food Sci, <u>35</u>, 95 (1970)".

Le seuil de perception olfactive dans l'eau du composé étudié est de $10^{-6}$ p.p.m.. On peut rapprocher l'impression percue de celle que décrit PATTERSON R.L.S. dans "Chemistry and Industry, 548 (1968)", à propos d'un composé chimiquement voisin, la mercapto-2 méthyl-2 pentanone-5 : une odeur d'urine de chat ou de feuille de cassissier froissée aux fortes dilutions, mais qui devient de plus en plus franchement désagréable au fur et à mesure que l'on augmente la concentration. STOFFELSMA J.H. et PIJPKER J.B., dans la demande de brevet allemand n°2.316.456, signalent que les composés répondant à la formule générale :

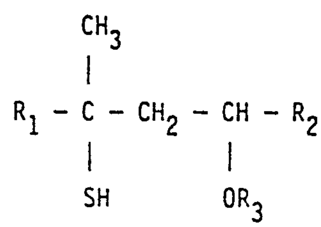

formule dans laquelle $R_1$ et $R_2$ ont diverses significations, $R_1$ pouvant représenter un groupement méthyle et $R_2$, un hydrogène, et $R_3$ représentant un hydrogène, un reste

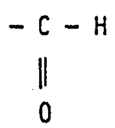 ou un reste 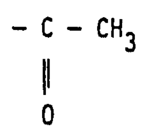 ,

présentent des propriétés olfactives intéressantes. Ces auteurs indiquent notamment que ces composés peuvent renforcer la note "Buchu" ou "bourgeon de cassis", dans des arômes de petits fruits ou des compositions synthétiques. Par ailleurs, LAMPARSKY D. et SCHUDEL P. dans "Tetrahedron. Lett., 36, 3323 (1971)" ont identifié, dans l'essence de Buchu, qui possède une note odorante cassis, un autre thiol tertiaire, le p-menthanone-3 thiol-8. Le composé de formule (I) selon la présente invention appartient donc à un groupe de thiols présentant des notes odorantes similaires.

Le Déposant a par ailleurs vérifié l'influence de l'incorporation du composé de formule (I) selon l'invention à une liqueur de cassis titrant 16°GL, préparée en liquoristerie, à partir d'un lot de baies et jugée peu aromatique par des experts. La liqueur, aromatisée avec le composé de formule (I) selon la présente invention a été proposée, avec addition de diverses concentrations du composé obtenu par synthèse, au jury qui avait établi le seuil de perception dans l'eau. Ce dernier détecta la présence du thiol à partir d'une teneur de $10^{-4}$ p.p.m., soit 100 fois plus que dans l'eau.

Par ailleurs, le Déposant a effectué un test hedonique portant sur l'addition de $2.10^{-4}$ p.p.m. du composé de formule (I) (soit 2 fois la valeur du seuil), à cette même liqueur. L'effet fut jugé bénéfique dans l'immédiat et il en a été de même après un mois de conservation des liqueurs à 25°C dans des flacons bouchés. Il semble donc bien que le composé de formule (I) soit le constituant majeur de l'arôme du bourgeon de cassis.

## REVENDICATIONS

1. Composé chimique représenté par la formule (I) :

$$\begin{array}{c} CH_3 \\ | \\ CH_3 - C - CH_2 - CH_2 - O - CH_3 \\ | \\ SH \end{array} \qquad (I)$$

2. Procédé de préparation du composé représenté par la formule (I) indiquée à la revendication 1, caractérisé par le fait que :

- dans une première étape, on prépare de l'huile essentielle de bourgeon de cassis ;
- dans une seconde étape, on réalise le fractionnement de ladite huile essentielle, par chromatographie liquide-solide, en au moins deux fractions, la première qui renferme les hydrocarbures mono- et sesquiterpéniques entrant dans la composition de ladite huile essentielle et que l'on obtient en utilisant, comme éluant, un milieu solvant apolaire, et la seconde, ou chacune des fractions suivantes, que l'on obtient en utilisant, comme éluant, respectivement un milieu solvant polaire, ou successivement des milieux solvants de polarité croissante ;
- dans une troisième étape, on réalise une première chromatographie en phase gazeuse d'au moins une partie de la (ou des) fraction(s) obtenue(s) à la seconde étape qui ne renferme(nt) pas lesdits hydrocarbures, en pratiquant au moins une injection dans l'appareil de chromatographie et en piégeant, à chaque fois, à la sortie dudit appareil, la portion où prédomine la note "urine de matou", afin de recueillir, après désorption du piège, à l'aide d'un milieu solvant à caractère polaire, un éluat renfermant le composé recherché ; et
- dans une quatrième étape, on réalise une seconde chromatographie en phase gazeuse d'au moins une partie de l'éluat obtenu à la troisième étape, en pratiquant au moins une injection dans l'appareil de chromatographie et en piégeant, à chaque fois, à la sortie dudit appareil, la portion correspondant au composé recherché, et on recueille ledit composé à l'état pur.

3. Procédé selon la revendication 2, caractérisé par le fait qu'à la seconde étape, on réalise le fractionnement de l'huile essentielle en trois fractions.

4. Procédé selon l'une des revendications 2 et 3, caractérisé par le fait qu'à la seconde étape, on utilise le pentane comme milieu solvant apolaire ; dans le cas où l'on obtient deux fractions, le dichlorométhane ou un mélange pentane-dichlorométhane comme milieu solvant polaire ; et, dans le cas où l'on obtient un nombre de fractions supérieur à deux (un ou des) mélange(s) pentane-dichlorométhane et le dichlorométhane comme milieux solvants de polarité croissante.

5. Procédé selon les revendications 3 et 4 prises simultanément, caractérisé par le fait que l'on utilise, comme milieu solvant pour obtenir la deuxième fraction, un mélange pentane-dichlorométhane, dans un rapport en volume de 80-20.

6. Procédé selon l'une des revendications 2 à 5, caractérisé par le fait qu'à la seconde étape, on utilise une colonne de chromatographie liquide-solide dont l'adsorbant est constitué par de la silice.

7. Procédé selon l'une des revendications 2 à 6, caractérisé par le fait qu'on utilise, à la troisième étape, le même milieu solvant polaire que celui utilisé à la seconde étape dans le cas où l'on obtient deux fractions, ou que l'un des milieux solvants utilisés à ladite seconde étape dans le cas où l'on obtient un nombre de fractions supérieur à deux.

8. Procédé selon l'une des revendications 2 à 7, caractérisé par le fait qu'à la première étape, on prépare l'huile essentielle de bourgeon de cassis en effectuant un entraînement à la vapeur d'eau, suivi d'une extraction au pentane.

9. Procédé de préparation du composé représenté par la formule (I) indiquée à la revendication 1, caractérisé par le fait que :

- dans une première étape, on fait réagir, en solution inerte et sous atmosphère inerte, le magnésium avec un halogénure d'alkyle de formule (II) :

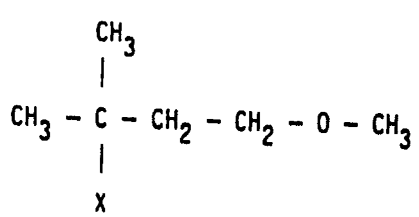

(II) ,

dans laquelle X est choisi parmi le chlore, le brome et l'iode, pour obtenir le composé organo-magnésien correspondant de formule (III) :

$$\begin{array}{c} \quad\quad CH_3 \\ \quad\quad | \\ CH_3 - C - CH_2 - CH_2 - O - CH_3 \\ \quad\quad | \\ \quad\quad MgX \end{array} \quad (III) ;$$

- dans une seconde étape, on fait réagir le soufre sur le composé de formule (III) pour obtenir le composé de formule (IV) :

$$\begin{array}{c} \quad\quad CH_3 \\ \quad\quad | \\ CH_3 - C - CH_2 - CH_2 - O - CH_3 \\ \quad\quad | \\ \quad\quad SMgX \end{array} \quad (IV) ; \text{ et}$$

- dans une troisième étape, on hydrolyse le composé de formule (IV) en milieu faiblement acide.

10. Procédé selon la revendication 9, caractérisé par le fait qu'on utilise un composé de formule (II) dans laquelle X est le chlore.

11. Procédé selon l'une des revendications 9 et 10, caractérisé par le fait qu'à la première étape, on prépare le composé de formule (III) en solution dans l'éther anhydre.

12. Procédé selon l'une des revendications 9 à 11, mettant en oeuvre un composé de formule (II), dans laquelle X est le chlore, caractérisé par le fait que l'on obtient ledit composé de formule (II) en faisant réagir l'isobutylène et le chlorométhyléther en présence de chlorure mercurique.

13. Utilisation du composé représenté par la formule (I) indiquée à la revendication 1, à titre d'arôme ou d'agent renforçateur d'arôme.

14. Composition destinée à être ingérée, caractérisée par le fait qu'elle renferme le composé représenté par la formule (I) indiqué à la revendication 1, à titre d'arôme ou d'agent renforçateur d'arôme.

15. Composition aqueuse selon la revendication 14, caractérisée par le fait que sa teneur minimale en composé de formule (I) est de l'ordre de

$10^{-6}$ p.p.m..

16. Liqueur selon la revendication 14, caractérisée par le fait que sa teneur minimale en composé de formule (I) est de l'ordre de $10^{-4}$ p.p.m..

FIG.1

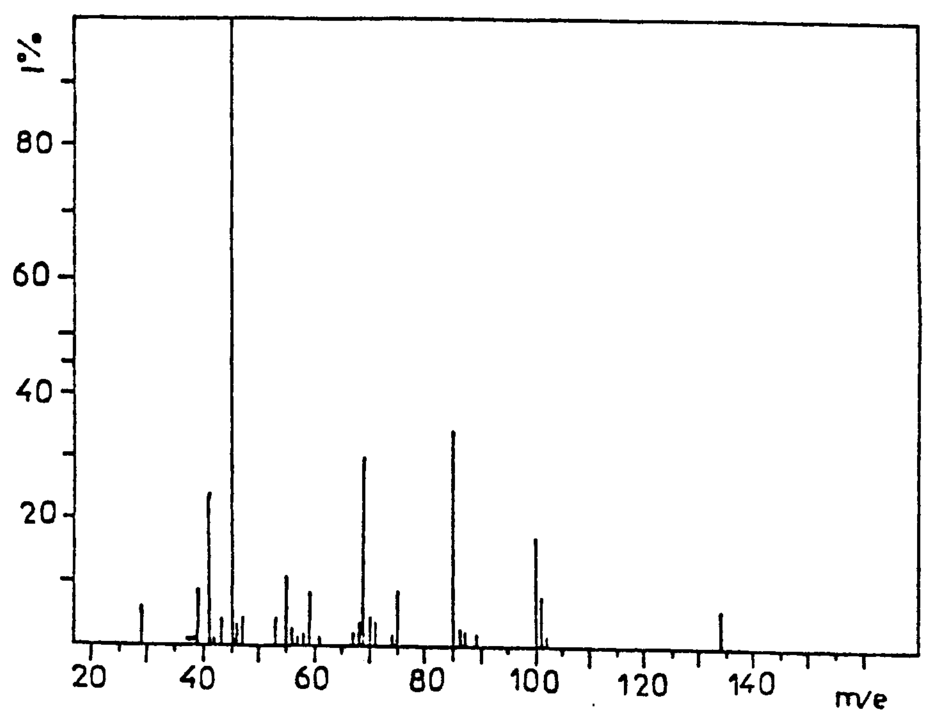

FIG. 2

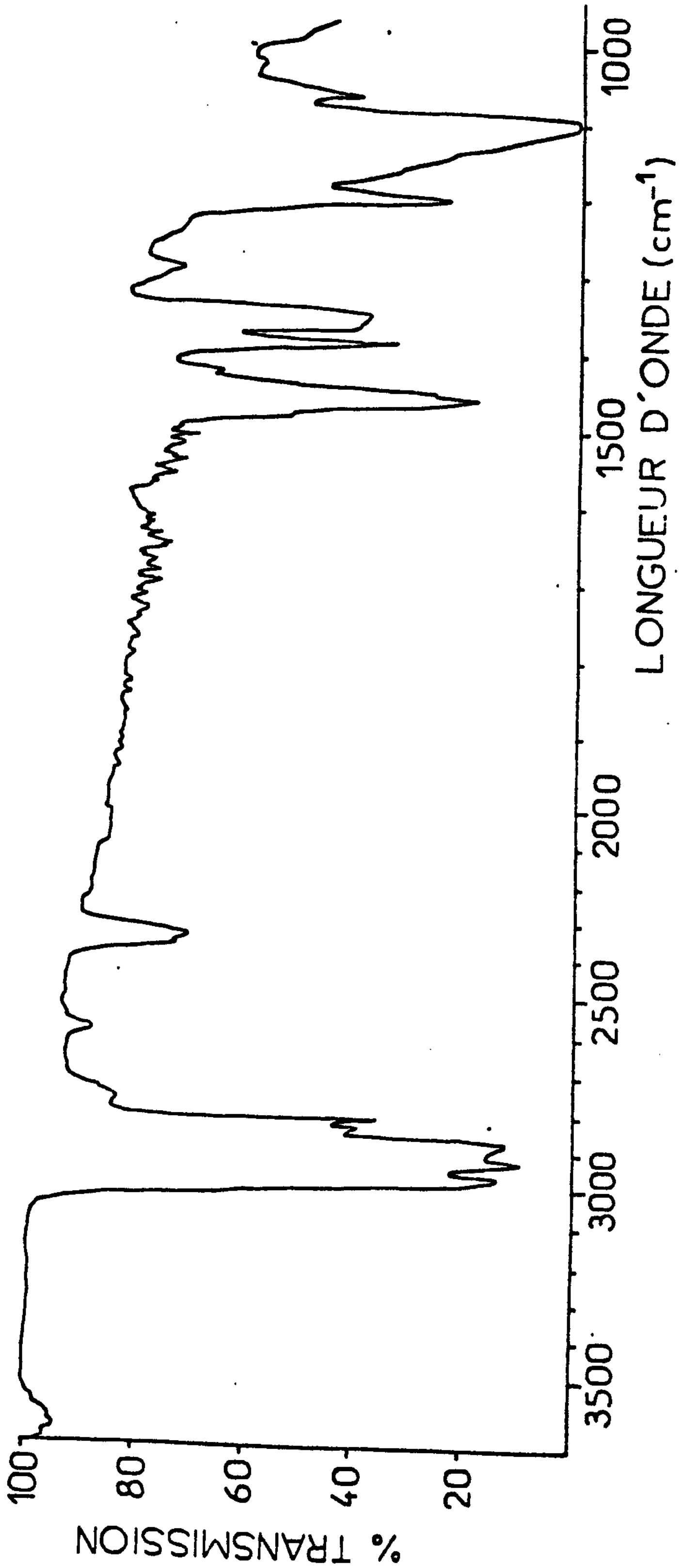
% TRANSMISSION
100
80
60
40
20
3500
3000
2500
2000
1500
1000
LONGUEUR D'ONDE ($cm^{-1}$)

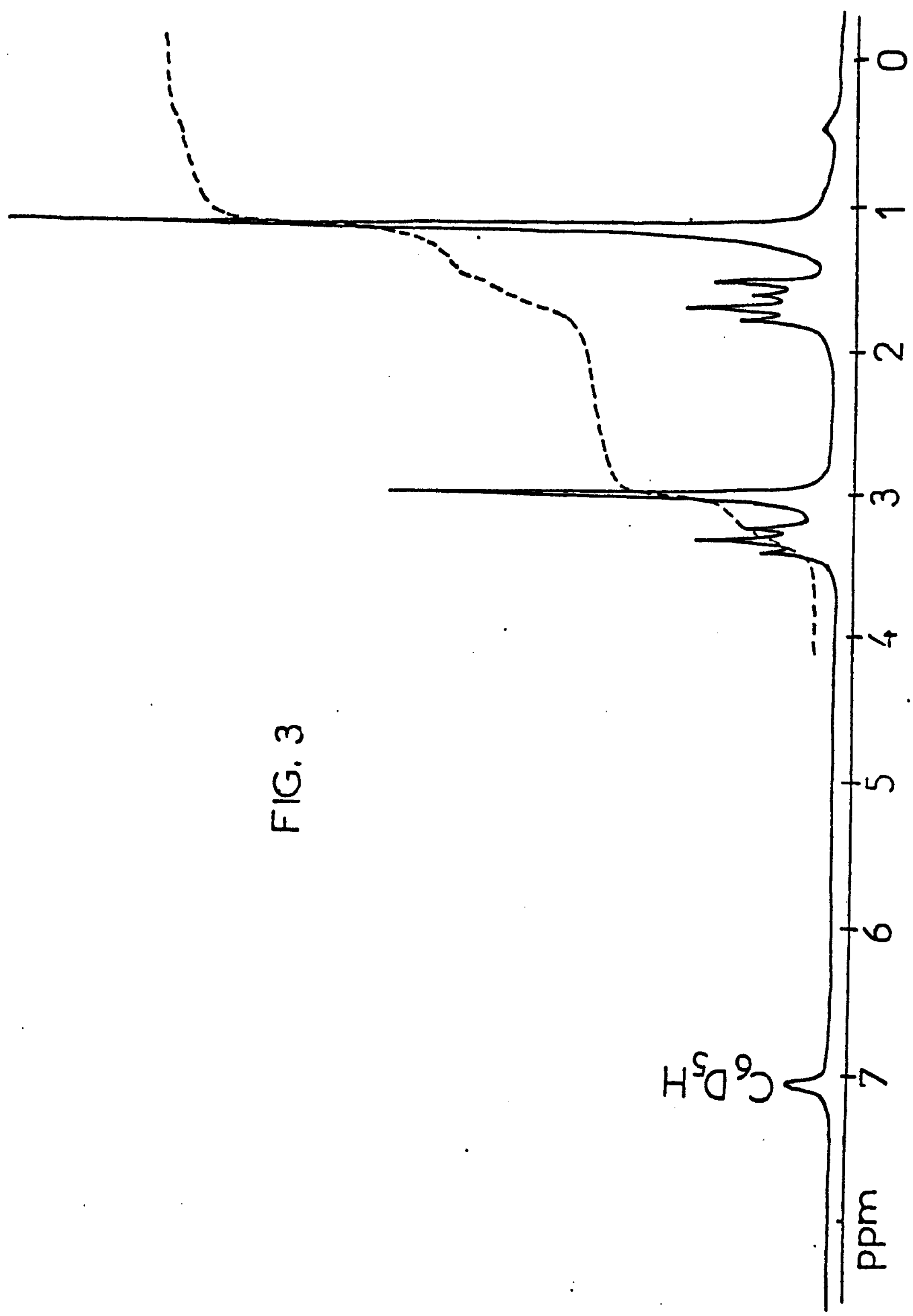


FIG. 3